# EUROPÄISCHE PATENTANMELDUNG

(11) **EP 4 644 524 A1**
(43) Veröffentlichungstag der Anmeldung: **05.11.2025**
(21) Anmeldenummer: 24173000.1
(22) Anmeldetag: 29.04.2024
(51) Int. Cl.: C12M 1/00, B01L 7/00, C12M 1/34, C12M 1/36

(54) **LABORGERÄT MIT INKUBATIONSKAMMERBELEUCHTUNG**

(71) Anmelder: Eppendorf SE, 22339 Hamburg (DE)
(72) Erfinder: Jolie, Christoph, Hamburg (DE); Fitzer, Jan, Ammersbek (DE); Stranzinger, Martin, Hamburg (DE)
(74) Vertreter: Wallinger Ricker Schlotter Tostmann

(57) **Zusammenfassung**

Die Erfindung betrifft ein Laborgerät mit Inkubationskammer und Beleuchtungseinrichtung zur Beleuchtung des Kammerinnenraums, die insbesondere hochtemperaturbeständig ist, um insbesondere bei beleuchtetem Kammerinnenraum ein Hochtemperaturdesinfektionsverfahren des Kammerinnenraums durchzuführen.

## Beschreibung

Die Erfindung betrifft ein Laborgerät mit beleuchtbarer Inkubationskammer, insbesondere einen Inkubator oder Laborschüttler für das Inkubieren von Mikroorganismen und Zellen, insbesondere von eukaryontischen Zellen. Die Erfindung betrifft zudem ein Verfahren zur Temperaturbehandlung in diesem Laborgerät.

Mit solchen inkubationsfähigen Laborgeräten, zum Beispiel Inkubatoren oder inkubierten Shakern, werden in biologischen und medizinischen Laboratorien Proben unter kontrollierten Umgebungsbedingungen gehalten. Temperatur und Gaszusammensetzung bzw. die Luftfeuchtigkeit der Atmosphäre im Inneren einer von der Umgebung isolierten Inkubatorkammer werden durch die apparativen Einrichtungen des Inkubators auf den gewünschten Werten gehalten. Die Kontrolle der Luftfeuchtigkeit im Inkubator ist entscheidend, da sie das Wachstum und die Entwicklung von Embryonen, Zellen oder Mikroorganismen beeinflusst. Unterschiedliche Arten von Organismen erfordern unterschiedliche Luftfeuchtigkeitsniveaus, daher ist es wichtig, die Feuchtigkeit möglichst präzise zu steuern, um optimale Bedingungen für die Inkubation zu gewährleisten. Inkubatoren für Zellen in Zellkultur ermöglichen das Wachstum lebender Zellen *in vitro* unter definierten atmosphärischen Bedingungen. Eukaryotische Zellen benötigen COz-Inkubatoren. Die Atmosphäre wird durch Luft mit einem bestimmten CO₂- und Oz-Gehalt und einer bestimmten Luftfeuchtigkeit gebildet, eine geeignete Temperatur ist oftmals 37 °C.

Der Innenraum eines Inkubators oder inkubierten Shakers muss mit einer hohen Luftfeuchte geregelt werden. Typisch sind relativ Luftfeuchtewerte um die 95%. Eine hohe und reproduzierbare Luftfeuchte ist wichtig, um zu verhindern, dass Medium aus inkubierten Zellkulturen verdunsten kann und somit Bestandteile aufkonzentriert werden. Reproduzierbarkeit ist eine Kernanforderung von Experimenten in der Zellkultur, so dass Bedingungen innerhalb des inkubierten Raumes immer reproduzierbar geregelt werden müssen. Ein weiterer wichtiger Aspekt ist die Gewährleistung von kondensatfreien Flächen im inkubierten Raum, um Kontaminationsbildung an diesen Stellen zu vermeiden.

Der Innenraum (Kammer) von Inkubatoren und inkubierten Shakern sollte zudem möglichst derartig ausgeführt sein, dass der Anwender die Oberflächen optimal reinigen kann. Zudem muss die gesamte Oberfläche so temperiert werden können, dass die Temperatur an keiner Stelle unter den Taupunkt fällt und eine Kondensatbildung vermieden wird, während die Homogenität der Lufttemperatur sehr genau sein muss, um Zellen überall in der Kammer optimal kultivieren zu können. Dennoch müssen in die Kammer verschiedene Schnittstellen gebracht werden, insbesondere Sensoren und Einlässe, um das optimale Klima für die Zellen gewährleisten zu können. Zum einen gilt das für die Temperatur und Feuchte und zum anderen aber auch für die Gasversorgung und die Kammerbeleuchtung. Gleichzeitig gilt es, die Anzahl an Schnittstellen zu minimieren, da diese auch immer schwieriger zu reinigen sind und über Schnittstellen auch Atmosphärenverluste (Gas; Luftfeuchte und Temperatur) eintreten.

Die meisten Kammern von Inkubatoren oder inkubierten Shakern ist nicht optimal zu reinigen und zu desinfizieren. Ein Anteil daran hat die Tatsache, dass alle Einlässe einen getrennten Port benötigen und diese Bereiche schlecht zu reinigen sind. Zudem erhöht sich die Gefahr von Kondensatbildung an diesen Stellen.

Der Erfindung liegt die Aufgabe zugrunde, ein einfach zu reinigendes Laborgerät mit beleuchtbarer Inkubationskammer bereitzustellen.

Die Erfindung löst die Aufgabe durch das Laborgerät gemäß Patentanspruch 1 und das Verfahren gemäß Patentanspruch 15 oder 16. Bevorzugte Ausgestaltungen dieser Gegenstände werden in den abhängigen Ansprüchen angegeben und lassen sich ferner der Beschreibung der Erfindung sowie den Zeichnungen entnehmen.

Da die Kammerwand im Betrieb des Laborgeräts auf gegenüber der Umgebung erhöhten Temperaturen liegt, lässt sich durch die Verwendung eines oder mehrerer Lichtleiter, die das Licht zur Kammer führen, die Positionierung der Lichtquelle(n) nahe der Kammerwand und damit die vorzeitige Alterung und ein Ausfall der Lichtquelle vermeiden. Dies erlaubt zudem die Hochtemperaturbehandlung (160-200°C) der beleuchtbaren/beleuchteten Kammer, was gegenüber bekannten Inkubatoren ein großer Vorteil ist. Zudem lässt sich die Lichtquelle gegenüber der Kammerwand an beliebiger Stelle flexibel anordnen, da der Lichtleiter flexibel und beliebig lang sein kann.

Für Beleuchtungszwecke im sichtbaren Spektrum, wie vorliegend, wird vorzugsweise ein Glasleiter als Lichtleiter, insbesondere Glasfasern, oder ein Kunststoff-Lichtleiter verwendet. Beide Arten von Lichtleitern eignen sich gut für die Übertragung von sichtbarem Licht.

Vorzugsweise ist der Lichtleiter ein stabförmiges, insbesondere starres, Bauteil. Er ist vorzugsweise hochtemperaturbeständig, insbesondere aus Borosilikatglas. Der Stab weist insbesondere einen Durchmesser zwischen 1 und 20 mm auf, vorzugsweise zwischen 2 und 10 mm, vorzugsweise zwischen 4 und 6 mm.

Der Lichtleiter kann auch biegbar sein, insbesondere eine Faser.

Glasfasern bestehen aus dünnen Glasfäden, die Licht durch Totalreflexion im Inneren des Glases führen. Diese Totalreflexion ermöglicht eine effiziente Lichtübertragung über große Entfernungen. Ein Glasfaserlichtleiter kann in Form von Lichtleiterkabeln verwendet werden, um Licht von einer oder mehreren Lichtquellen zu verschiedenen Eintrittsöffnungen oder einen einzigen Eintrittsöffnung zu führen.

Kunststoff-Lichtleiter bestehen insbesondere aus transparenten Kunststoffen wie Polymethylmethacrylat (PMMA) oder Polycarbonat. Diese Lichtleiter sind vorzugsweise flexibel. Sie funktionieren wie Glasfasern, indem sie Licht durch Totalreflexion im Inneren des Kunststoffs führen.

Vorzugsweise werden der eine oder mehrere Lichtleiter mit einer oder mehreren Lichtquellen, vorzugsweise LEDs (Light Emitting Diodes) oder Halogenlampen gekoppelt, um das Licht in den Lichtleiter einzuspeisen. An den Enden der Lichtleiter können verschiedene Arten von Optiken verwendet werden, um das Licht zu verteilen oder zu fokussieren, je nach den Anforderungen der jeweiligen Anwendung. Bevorzugt ist auch, keine Optik zu verwenden sondern ein planares, insbesondere abgebrochenes, insbesondere unpoliertes Ende des Lichtleiters zu verwenden, da die Qualität der Lichtemission in die Kammer auf diese Weise zufriedenstellend ist.

Der Lichtleiter kann ein zur Lichtquelle separates Bauteil sein, kann aber auch in das die Lichtquelle bildende Bauteil integriert bzw. integral mit diesem gebildet sein, insbesondere im Fall einer LED. Eine Lichtquelle kann insbesondere ein lichtemittierendes Bauteil aufweisen, insbesondere ein Halbleiterbauteil oder eine Platine, das/die von einer, insbesondere transparenten, Schutzeinrichtung eingehüllt ist. Die Schutzeinrichtung kann durch den Lichtleiter gebildet sein oder diesen aufweisen.

Insbesondere bei geeigneter Beschaffenheit, insbesondere einer Hochtemperaturresistenz (geeignet um die Hochtemperaturprogramme zum Zwecke der Desinfizierung des Kammerinnenraums zu tolerieren) der Lichtquelle, und/oder bei geeignetem Abstand der Lichtquelle an/von der Kammerwand kann die, insbesondere ein Beleuchtungsszenario realisierende Beleuchtungseinrichtung ohne den Lichtleiter vorgesehen sein, was zu einer alternativen Erfindung gehört. Die Beleuchtungseinrichtung weist dann mindestens eine Lichtquelle auf, die dazu angeordnet ist, Licht in den Innenraum der Inkubationskammer zu emittieren. Die weitere Gestaltung des Laborgeräts und dessen (lichtleiterfreie) Beleuchtungseinrichtung entspricht den hier vorzufindenden Beschreibungen dieser Komponenten und Subkomponenten, in all ihren möglichen Ausgestaltungen.

Ein Lichtleiter wird als eine Struktur oder ein Medium verstanden, die/das Licht durch interne Reflexion oder Brechung übertragen kann.

Jede oder mindestens eine der mindestens einen Lichtquelle ist vorzugsweise eine LED. Sie leistet vorzugsweise 2 Watt bis 20 Watt, vorzugsweise 5 bis 10 Watt. Der Lichtstrom liegt vorzugsweise bei 50 - 1000 Lumen, insbesondere 80 - 300 Lumen. Vorzugsweise ist im Laborgerät nur genau eine Lichtquelle zur Beleuchtung des Kammerinnenraums vorgesehen. Vorzugsweise ist im Laborgerät nur genau ein Lichtleiter zur Beleuchtung des Kammerinnenraums vorgesehen.

Vorzugsweise ist die mindestens eine Lichtquelle nicht an einer Kammerwand montiert. Da elektronische Lichtquellen, insbesondere LED's, keine hohen Temperaturen (größer als 100 Grad Celsius) vertragen, sondern insbesondere maximal, 60 °C, ist die Wärmeübertragung von der Kammerwand auf die LED zu vermeiden. Insbesondere befindet sich vorzugsweise keine Wärmebrücke mit/aus metallischem Material oder Material mit vergleichbar hoher Wärmeleitung zwischen Lichtquelle und Kammerwand. Ist eine Halteeinrichtung zur Halterung der Lichtquelle an der Kammerwand, insbesondere in einem Abstand zu dieser, vorgesehen, besteht diese vorzugsweise aus einem Material mit geringerer Wärmeleitung als Metall, vorzugsweise ist dieses Material Kunststoff, und/oder weist eine Wärmeleitfähigkeit kleiner als 15 W/(m*K) auf.

Vorzugsweise ist die mindestens eine Lichtquelle in einem Abstand d zur Kammerwand montiert, wobei d>0 , vorzugsweise 3 cm < d, vorzugsweise 5 cm < d. Dadurch wird die thermische Entkopplung und der Schutz der Lichtquelle weiter verbessert. Der Abstand wird insbesondere senkrecht zur Kammerwand gemessen. Aufgrund typischer Gehäuseabmessungen ist typischer Weise d < 20 cm oder d < 30 cm.

Vorzugsweise ist eine Lichtquelle in einem Abstand (d) zu einer Kammerwand angeordnet, und ein Lichtleiter erstreckt sich vorzugsweise ausgehend von der Lichtquelle bis zu einer Eintrittsöffnung, und/oder verläuft insbesondere entlang einer Längsachse (A), und/oder insbesondere senkrecht zur Kammerwand. Dadurch wird das Licht bei maximiertem Abstand effizient zur Eintrittsöffnung geführt.

Vorzugsweise weist die Beleuchtungseinrichtung eine Halteeinrichtung auf, die den Lichtleiter relativ zur Eintrittsöffnung in einer Montageposition hält. Dadurch wird der Lichtleiter geschützt und der zuverlässige Betrieb gewährleistet. Die Halteeinrichtung weist insbesondere mindestens ein Haltemittel auf, mit dem der Lichtleiter an der Eintrittsöffnung gehalten wird. Dadurch wird eine zuverlässige Einkopplung des Lichts in den Kammerinnenraum erreicht.

Vorzugsweise weist ein Material oder weisen alle Materialien der Halteeinrichtung eine Wärmeleitfähigkeit kleiner als 15 W/(m*K) auf. Insbesondere besteht die Halteeinrichtung aus einem oder mehreren Kunststoffen. Durch die geringe Wärmeleitfähigkeit wird vermieden, dass zu viel Wärme von der Kammer zur Lichtquelle gelangt, und umgekehrt, dass zu viel Wärme von der Kammer weg und über die Halteeinrichtung in die Umgebung geführt wird.

Vorzugsweise ist entlang der Außenseite der Kammerwand eine thermische Isolationsschicht angeordnet, die insbesondere eine Öffnung aufweist, durch die sich der Lichtleiter bis zur Eintrittsöffnung erstreckt. Dadurch wird die Lichtquelle weiter vor einer Überhitzung durch Wärmeübertragung von der Kammer geschützt.

Vorzugsweise weist die Halteeinrichtung ein Haltemittel auf, das mit der thermischen Isolationsschicht verbunden ist, insbesondere formschlüssig und/oder kraftschlüssig verbunden ist, optional auch stoffschlüssig. Vorzugsweise weist die Halteeinrichtung ein thermisch isolierendes Bauteil auf, insbesondere das Anschlusselement, oder insbesondere einen Isolationsstopfen, der die Öffnung in der thermischen Isolationsschicht verschließt und so eine Konvektionssperre bildet.

Vorzugsweise weist das Laborgerät mindestens ein Gasleitungselement auf, das in die Eintrittsöffnung der Kammerwand mündet, in die auch der Lichtleiter sein Licht leitet, so dass ein aus dem Gasleitungselement strömendes Gas durch die (Licht-)Eintrittsöffnung in den Kammerinnenraum gelangt. Auf diese Weise kann auf eine zusätzliche Kammerwandöffnung für die Gaszuleitung verzichtet werden. Jede unnötige Öffnung der Kammerwand ist grundsätzlich unerwünscht, um Kontamination und Atmosphärenverlust in der Kammer gering zu halten.

Vorzugsweise verlaufen der Lichtleiter und das Gasleitungselement entlang eines gemeinsamen Pfades, der linear und/oder gekrümmt sein kann, und münden vorzugsweise in derselben Eintrittsöffnung.

Vorzugsweise verlaufen der Lichtleiter und das Gasleitungselement koaxial. Sie können aber auch nicht-koaxial oder insbesondere nebeneinander verlaufen.

Vorzugsweise verläuft der Lichtleiter innerhalb des Gasleitungselements und mündet vorzugsweise in einer Auslassöffnung des Gasleitungselements. Der Lichtleiter kann dabei aus der Auslassöffnung des Gasleitungselements in die Kammer herausragen, oder das Gasleitungselement kann über das Ende des Lichtleiters hinausragen. Das Ende des Lichtleiters fluchtet aber vorzugsweise im Wesentlichen mit dem Ende des Gasleitungselements.

Vorzugsweise wird der Lichtleiter form- und/oder kraftschlüssig in dem Gasleitungselement, insbesondere in einem verengten Abschnitt eines zylinderförmigen Hohlraums des Gasleitungselements oder in der Auslassöffnung gehalten.

Vorzugsweise weist ein Innenraum des Gasleitungselements, insbesondere die Auslassöffnung, mindestens einen radial nach innen weisenden Vorsprung auf, vorzugsweise drei oder mehr solcher Vorsprünge, an dem sich der Lichtleiter abstützt. Zwischen den Vorsprüngen verbleibt insbesondere jeweils ein Durchgangskanal für das Gas.

Vorzugsweise ist das Gasleitungselement ein hohlzylinderförmiges Bauteil, in der der Lichtleiter eingeführt ist, insbesondere an einer Stirnseite des hohlzylinderförmigen Bauteils. Vorzugsweise ist das Gasleitungselement ein Bestandteil der Halteeinrichtung.

Vorzugsweise weist die Beleuchtungseinrichtung ein Anschlusselement auf. Das Anschlusselement ist insbesondere dazu eingerichtet, eine Lichtquelle, insbesondere LED oder Platine mit LED, an den Lichtleiter anzuschließen. Vorzugsweise ist mindestens eine Lichtquelle angeordnet, um Licht in eine Stirnseite des Lichtleiters einzukoppeln. Das Anschlusselement ist insbesondere dazu eingerichtet, eine Gaszuleitung an das Gasleitungselement anzuordnen, wenn dieses ein Bestandteil einer als kombinierten Gas-/Lichtführungseinrichtung ausgebildeten Beleuchtungseinrichtung ist. Das Anschlusselement ist insbesondere Teil der Halteeinrichtung. Es weist vorzugsweise einen Aufnahmeraum auf, in den ein stromaufwärts gelegenes Ende des Gasleitungselements mündet, und in den ein stromabwärts gelegenes Ende einer Gasanschlussleitung und/oder Gaszuleitung mündet und durch den insbesondere der Lichtleiter verläuft.

Vorzugsweise weist das hohlzylinderförmige Bauteil, insbesondere am stromabwärts gelegenen Ende, einen Flansch auf, der insbesondere Teil der Halteeinrichtung ist, und der insbesondere innerhalb der Kammer angeordnet ist und dort insbesondere an der Eintrittsöffnung anliegt, während sich vorzugsweise ein hohlzylinderförmiger Abschnitt des hohlzylinderförmigen Bauteils durch die Eintrittsöffnung nach außen erstreckt.

Vorzugsweise ist ein Lichtleiter ein Glasstab oder weist diesen auf, und der Glasstab besteht insbesondere aus Borosilikatglas.

Die Erfindung betrifft auch ein Verfahren zur thermischen Behandlung des Kammerinnenraums eines erfindungsgemäßen Laborgeräts zu Desinfektionszwecken, das eine elektrische Steuereinrichtung aufweist, die dazu programmiert ist, ein Hochtemperaturprogramm (auch bezeichnet als Hochtemperier- oder Hochtemperatur: - programm, -vorgang, -verfahren) auszuführen, durch das der beleuchtbare Kammerinnenraum mittels der Heizeinrichtung für einen vorbestimmten Zeitraum auf eine vorbestimmte Temperatur aufgeheizt wird, wobei der Zeitraum zwischen 1 Minute und 12 Stunden betragen kann, und wobei die Temperatur zwischen 90 °C und 200°C betragen kann, insbesondere zwischen 120 °C und 200 °C, bevorzugt zwischen einschließlich 170°C und 200°C, und besonders bevorzugt zwischen 180°C und 200°C wobei das Verfahren den Schritt aufweist:
- Aufheizen des beleuchtbaren, insbesondere mittels der Beleuchtungseinrichtung beleuchteten, Kammerinnenraums mittels der Heizeinrichtung für einen vorbestimmten Zeitraum auf eine vorbestimmte Temperatur, wobei der Zeitraum zwischen 1 Minute und 12 Stunden betragen kann, und wobei die Temperatur zwischen 90 °C und 200°C betragen kann, insbesondere zwischen 120 °C und 200 °C, bevorzugt zwischen einschließlich 170°C und 200°C, und besonders bevorzugt zwischen einschließlich 180°C und 200°C, betragen kann. Eine Temperatur von 170° bzw. 180°C und höher ist besonders vorteilhaft zur Desinfektion und eine in diesem Temperaturbereich gereinigte Kammer wird bevorzugt vom Anwender eingesetzt für die Kultivierung von eukaryontischen Zellen, deren Verlust durch Kontamination ein hochgradiges Forschungsrisiko darstellt.

Das Laborgerät weist vorzugsweise eine Heizeinrichtung auf, die zum Beheizen der Kammer vorgesehen und insbesondere zum Beheizen der Kammer eingerichtet ist. Die Heizeinrichtung weist mindestens einen Heizwendel auf, der an der Außenseite der Kammerwände verlegt ist, insbesondere im thermischen Kontakt mit dieser Außenseite. Die Heizwendel funktionieren aufgrund des elektrischen Widerstandsphänomens. Wenn elektrischer Strom durch einen Leiter mit Widerstand fließt, erzeugt dies Wärme. Die Heizwendel bestehen vorzugsweise aus Material mit hoher elektrischer Widerstandsfähigkeit, wie zum Beispiel Nichrom (Nickel-Chrom-Legierung), die bei Durchführung eines elektrischen Stroms heiß werden. Die Funktion einer Heizwendel beruht auf dem Prinzip des Joule'schen Gesetzes, das besagt, dass die Erwärmung eines Leiters proportional zum Quadrat des durch ihn hindurch fließenden Stroms und zum elektrischen Widerstand des Leiters ist. Durch das Anlegen einer elektrischen Spannung an die Heizwendel fließt ein Strom durch sie hindurch, was zu Wärmeentwicklung führt.

Die Erfindung betrifft auch ein Laborgerät zur Inkubation von in Probengefäßen (130) enthaltenen flüssigen Laborproben, insbesondere Inkubationsschüttler, aufweisend
- eine Kammer (2), die mindestens eine Kammerwand aufweist und eine Kammeröffnung (2a) zum Einstellen und Herausnehmen der Probengefäße (130) in einen Innenraum (3) der Kammer,
- eine Heizeinrichtung (190), die zum Beheizen der Kammer vorgesehen ist, die mindestens einen Heizwendel aufweist, der an der Außenseite der mindestens einen Kammerwand angeordnet ist,
dadurch gekennzeichnet, dass
- die mindestens eine Kammerwand mindestens einen ersten Flächenbereich aufweist, in dem die von dem mindestens ein Heizwendel abgegebene Heizleistung größer ist als in einem zweiten Flächenbereich, insbesondere indem der mindestens ein Heizwendel im ersten Bereich mit einer höheren Flächendichte verlegt ist.

Die Heizleistung wird in Watt angegeben. Sie kann bei einem Heizdrahtabschnitt über eine elektrische Messung gemessen werden. Der bei diesen Angaben zugrunde gelegte Flächenbereich beträgt in der Praxis insbesondere zwischen 10 und 50 Quadratdezimeter.

Bei der Inkubationskammer liegen die senkrechten Abstände von parallel verlegten Drähten in den zentralen Flächenbereichen (zweite Flächenbereiche) der Boden-, Seiten, Rück- und Deckenwand zwischen 3 und 15 cm, insbesondere zwischen 4 und 10 cm. In den Randbereichen und nahe von Öffnungen der Kammer (erste Flächenbereiche) sind die Abstände vorzugsweise geringer als in den zentralen Flächenbereichen.

Vorzugsweise ist die Wärmestromdichte, angegeben in Watt/Quadratmeter, in dem ersten Flächenbereich größer als in dem zweiten Flächenbereich.

Der Heizdraht bzw. Heizwendel ist insbesondere auf die Kammerwand geklebt, insbesondere mittels eines Klebebands, insbesondere metallischen Klebebands, insbesondere Aluminiumklebeband.

Vorzugsweise ist der Flächenbelegungsanteil durch den mindesten einen Heizwendel, also die vom Heizwendel auf der Oberfläche belegte Fläche A_H geteilt durch die Flächeneinheit A, also A_H / A, in dem ersten Flächenbereich größer als in dem zweiten Flächenbereich.

Vorzugsweise ist die pro Flächeneinheit auf der Oberfläche verlegte Länge des mindestens einen Heizwendel, gemessen in Meter pro Quadratmeter, in dem ersten Flächenbereich größer als in dem zweiten Flächenbereich.

Bevorzugt ist jeweils, den Anteil der Länge von Heizdraht pro planarer Fläche (zum Beispiel zentral im Deckenwandbereich der Kammer) -als zweitem Flächenbereich - mit dem Anteil der Länge von Heizdraht im Kammerwandrandbereich und/oder einer Kammerwandöffnung und/oder einem Kammerwandkrümmungsbereich -als erstem Flächenbereich- vergleichen.

Vorzugsweise liegt der erste Flächenbereich näher an einem Rand der Kammerwand, einer Öffnung der Kammerwand und/oder einem Krümmungsbereiche, insbesondere einer Ecke, der Kammer, als der zweite Flächenbereich. In den genannten Bereichen wird im Vergleich zur planaren Fläche der Kammerwand mehr Wärme an die Umgebung abgeführt, was durch die höhere Heizwendeldichte bzw. höhere Heizleistung kompensierbar ist. Im Resultat wird eine homogenere Kammertemperatur erreicht und das Risiko der Kondensatbildung an insbesondere den ersten Flächenbereichen vermieden.

Vorzugsweise weist das Laborgerät eine elektrische Steuereinrichtung auf, insbesondere eine Datenverarbeitungseinrichtung, und ist vorzugsweise dazu programmiert, eine Temperatur einer Kammer, insbesondere der Kammerwand, zu erfassen und insbesondere in Abhängigkeit von dieser Temperatur die Leistung der Heizeinrichtung einzustellen. Vorzugsweise ist die Steuereinrichtung dazu programmiert, eine Temperatur der Heizeinrichtung, auf eine gewünschte, insbesondere konstante, Zieltemperatur zu regeln. Vorzugsweise ist die Steuereinrichtung dazu programmiert, einen Heizregelkreis zu bilden, der dazu eingerichtet ist, die mittels eines Temperatursensors gemessene Temperatur eines Heizelements des Verdampfers auf eine konstante Zieltemperatur zu regeln, bei der ein mit dem Heizelement in Kontakt kommendes Wasservolumen verdampft wird und dabei dem Heizelement Wärme entzieht,

Die elektronische Steuereinrichtung ist vorzugsweise dazu programmiert, mindestens eine Funktion der Beleuchtungseinrichtung, die insbesondere auch keinen Lichtleiter aufweisen kann, zu steuern, insbesondere die Dauer und/oder Intensität und/oder Farbe des eingestrahlten Lichts und/oder in Abhängigkeit von Sensorsignalen, insbesondere dem Signal eines Türsensors des Inkubators, um so unterschiedliche Beleuchtungsszenarien zu erreichen. Insbesondere ist die Steuereinrichtung dazu eingerichtet, die Beleuchtungseinrichtung über eine Benutzerschnittstelle von einem Nutzer bedienbar zu machen. So sind vorzugsweise verschiedene Beleuchtungsmodi, die sich in der Lichteinstrahlungsdauer und/oder Lichtintensität und/oder der Lichtfarbe unterscheiden von einem Nutzer, insbesondere über eine grafische Benutzeroberfläche (GUI) der Benutzerschnittstelle, mittels der Benutzerschnittstelle auswählbar. Die Verwendung verschiedener Beleuchtungsmodi bzw. Beleuchtungsszenarien ist von großem Vorteil für den Nutzer des Laborgerätes mit der erfindungsgemäßen Beleuchtungsvorrichtung, da so dem Nutzer bereits durch eine Glastür bzw. ein Glasfenster des Gerätes Hinweise über den Status des Gerätes gegeben werden können. Üblicherweise verfügen Laborgeräte zur Inkubation von Proben (CO₂, Inkubatoren, Inkubationsshaker und Shaker) über eine innere Glastür oder eine Tür mit Fenster. Die elektronische Steuereinrichtung ist vorzugsweise dazu programmiert, mindestens einen Beleuchtungsparameter zu steuern, insbesondere zu variieren.

Die elektronische Steuereinrichtung ist vorzugsweise dazu programmiert, mindestens einen die Beleuchtung kennzeichnenden Beleuchtungsparameter der Beleuchtungseinrichtung, die insbesondere auch keinen Lichtleiter aufweisen kann, zu steuern, wobei der/die Beleuchtungsparameter ausgewählt sein kann/können aus der Liste der Beleuchtungsparameter, die das von der Beleuchtungseinrichtung in die Kammer eingestrahlte Licht kennzeichnen: die Dauer der Lichteinstrahlung, die Farbe des eingestrahlten Lichts, der Zeitverlauf der Lichteinstrahlung, insbesondere wechselnde Intensität und/oder wechselnde Farben, insbesondere in Form einer blinkenden, insbesondere pulsierenden Beleuchtung, oder in Form eines Fadings. Der Zeitverlauf kann vorzugsweise so sein, dass die Intensität des eingestrahlten Lichts mit der Zeit erhöht wird ("fade in"). Der Zeitverlauf kann vorzugsweise so sein, dass die Intensität des eingestrahlten Lichts mit der Zeit reduziert wird ("fade out"). Ein oder mehrere Beleuchtungsparameter können das von der Beleuchtungseinrichtung emittierte Licht prägen.

Die elektronische Steuereinrichtung ist vorzugsweise dazu programmiert, mindestens einen Beleuchtungsparameter in Abhängigkeit von mindestens einem Beleuchtungssteuerparameter auszuwählen und einzustellen.

Der Beleuchtungssteuerparameter kann ein Sensorsignal sein. Ein "fade in" kann insbesondere programmiert sein für den Fall, dass die Steuereinrichtung mittels eines Türsensors des Laborgeräts ein Öffnen der Türe detektiert. Ein "fade out" kann insbesondere programmiert sein für den Fall, dass die Steuereinrichtung mittels eines Türsensors des Laborgeräts ein Schließen der Türe detektiert. Das Sensorsignal kann auch von einem Gassensor stammen, insbesondere CO2-Sensor, rH-Sensor (Luftfeuchte Sensor), Temperatursensor. Die elektronische Steuereinrichtung kann dazu programmiert sein, die Zuführung von CO2-Gas, Wasserdampf, einem anderen Gas, oder eine Heizaktivität der (Kammer)-Temperiereinrichtung durch Ändern des Beleuchtungsparameters anzuzeigen, insbesondere durch ein Blinken oder eine für den Vorgang charakteristische Farbe anzuzeigen.

Die elektronische Steuereinrichtung kann dazu programmiert sein, eine Abweichung eines physikalischen Parameters von seinem Referenzwert, insbesondere dem Referenzwert zuzüglich eines Toleranzbereichs, durch eine Änderung eines Beleuchtungsparameters anzuzeigen. Der physikalische Parameter kann die Inkubatoratmosphäre kennzeichnen und kann gewählt sein aus der Gruppe von Parametern: Gaskonzentration, insbesondere CO2-Gaskonzentration in der Kammer, gemessen durch einen Gassensor des Laborgeräts, insbesondere CO2-Sensor; Wasserdampfkonzentration in der Kammer, gemessen durch einen rH-Sensor des Laborgeräts, Temperatur der Inkubatoratmosphäre oder der Kammer, gemessen durch einen Temperatursensor in oder an der Kammer. Der Referenzwert kann der jeweilige Sollwert sein, insbesondere CO2-Konzentrations-Sollwert, Luftfeuchtesollwert, Temperatursollwert. Diese können vom Benutzer über eine Benutzerschnittstelle des Laborgeräts eingegeben sein und/oder können in einem Datenspeicher des Laborgeräts gespeichert sein.

Der Beleuchtungssteuerparameter kann ein vom Benutzer oder vom Programm gesetzter Geräteparameter sein, insbesondere ein Gerätestatusparameter. Ein Gerätestatusparameter kann Fehlercodes kennzeichnen oder kann den ordnungsgemäßen Betriebszustand kennzeichnen. Ein Gerätestatusparameter kann die Aktivität eines Hochtemperaturprogramms kennzeichnen, oder mindestens eine Temperaturstufe eines Hochtemperaturprogramms (z.B. eine rote Beleuchtung während der Ausführung eines Hochtemperaturprogramms). Ein Fehlercode kann beinhalten, dass eine Abweichung eines physikalischen Parameters von seinem Referenzwert erfasst wurde, insbesondere dem Referenzwert zuzüglich eines Toleranzbereichs, wie oben beschrieben. Ein Fehlerode kann auch auf Parameter bezogen sein, die nicht die Inkubatoratmosphäre kennzeichnen, vorzugsweise: einen Zeitparameter, insbesondere Ablauf eines Timers oder Erreichen eines mittels Zeitgebers gemessenen Datums oder Uhrzeit, wobei der Zeitparameter insbesondere ein vom Hersteller oder Benutzer vorgegebenes Wartungsintervall kennzeichnen kann, eine Gesamtbetriebsdauer, eine Zeit zwischen Hochtemperiervorgängen, die in der Kammer zu Desinfektionszwecken durchgeführt werden. Ein möglicher Geräteparameter wäre auch ein mittels Sensorsignal ermittelter Parameter, oder ein Parameter, der in einer Datenspeichereinrichtung des Laborgeräts gespeichert ist.

Die Funktionen der Steuereinrichtung sind insbesondere durch Programmcode und/oder durch elektronische Schaltkreise implementiert. Die Steuereinrichtung kann einen Mikrocontroller, eine Recheneinheit (CPU) zum Verarbeiten von Daten bzw. einen Mikroprozessor aufweisen, die jeweils der Datenverarbeitungseinrichtung zugeordnet sein können.

Die Steuereinrichtung kann als selbständig arbeitendes Bauteil ausgebildet sein, das die Funktionen der Beleuchtungseinrichtung steuert, das aber insbesondere nicht eine oder mehrere Funktionen des Laborgeräts steuert, mit dem die Beleuchtungseinrichtung verbunden ist oder dessen Bestandteil die Beleuchtungseinrichtung vorzugsweise ist.

Die Steuereinrichtung kann aber auch durch eine solche Steuereinrichtung gebildet sein, die außer den Funktionen der Beleuchtungseinrichtung auch mindestens eine, mehrere oder alle Funktionen des Laborgeräts steuert, mit dem die Beleuchtungseinrichtung verbunden ist oder dessen Bestandteil die Beleuchtungseinrichtung vorzugsweise ist. Eine der Funktionen des Laborgeräts ist insbesondere die Regelung der Temperatur in der Inkubationskammer des Laborgeräts, oder die Regelung der Gaszusammensetzung in der Inkubationskammer, insbesondere der CO₂-Konzentration. Eine der Funktionen des Laborgeräts ist insbesondere auch die Steuerung eines Benutzerschnittstellenmoduls des Laborgeräts, das dem Benutzer Informationen anzeigt, insbesondere über Sensorwerte zu in/an der Inkubationskammer gemessenen physikalischen oder chemischen Größen.

Das Laborgerät ist vorzugsweise ein Laborinkubator. Ein Inkubator bezeichnet insbesondere ein Laborgerät mit einer Inkubatorkammer (Inkubationskammer), deren Atmosphäre (Gas, insbesondereCOz, und Luftfeuchte) vom Inkubator auf eine vorgegebene Zieltemperatur regelbar ist bzw. geregelt wird. Insbesondere handelt es sich um ein Laborgerät, mit dem kontrollierte Klimabedingungen für verschiedene biologische Entwicklungs- und Wachstumsprozesse geschaffen und erhalten werden können. Der Inkubator kann ein Schüttelinkubator (Shaker), also ein Inkubator mit einer Bewegungseinrichtung zur Bewegung von in der Inkubatorkammer angeordneten Objekten, sein oder diesen beinhalten, und kann ein Microbial-Inkubator (auch ohne CO₂) sein. Der Inkubator kann insbesondere als eine Zellkultivierungsvorrichtung ausgebildet sein. Der Inkubator dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO₂ und/oder des O₂ und/oder des N₂-Gehalts des Gases und/oder eine Einstelleinrichtung zur Einstellung der Luftfeuchtigkeit in der Inkubatorkammer des Inkubators.

Das Laborgerät, bzw. der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. Je nach Ausführungsform kann darüber auch die Luftfeuchte geregelt werden.

COz-Inkubatoren dienen insbesondere der Kultivierung tierischer bzw. humaner Zellen.

Solche Laborgeräte können Wendevorrichtungen zum Wenden des mindestens einen Zellkulturbehälters und/oder eine Schütteleinrichtung zum Schütteln bzw. Bewegen des mindestens einen Zellkulturbehälters aufweisen. Der erfindungsgemäße Inkubator ist insbesondere nicht ein Bioreaktor oder Fermenter.

Das Laborgerät, bzw. der Inkubator, kann mindestens eine Sensoreinrichtung aufweisen. Eine Sensoreinrichtung weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an CO₂ und/oder O₂ und/oder N₂. Eine Sensoreinrichtung weist vorzugsweise einen Sensor zur Ermittlung der relativen Luftfeuchtigkeit auf.

Das Laborgerät, bzw. der Inkubator, weist vorzugsweise eine bzw. eine einzige Inkubatorkammer auf. Diese kann in Kompartimente unterteilt sein. Kompartimente können durch - insbesondere gelochte- Lagerplatten getrennt sein, wobei insbesondere ein Gasaustausch zwischen den Kompartimenten ermöglicht ist.

Die Inkubatorkammer (auch bezeichnet als Kammer oder Inkubationskammer) weist Kammerwände bzw. Kammerinnenwände und genau eine oder mindestens eine Kammeröffnung auf, über die die Objekte bzw. Zellkulturbehälter im Inneren der Inkubatorkammer platzierbar und entnehmbar sind. Die Kammeröffnung ist vorzugsweise durch ein mit der Inkubatorkammer oder einem anderen Inkubatorabschnitt beweglich verbundenes Verschlusselement verschließbar, insbesondere eine mittels Scharnier oder Schwenktüre (vorzugsweise: "slide-up door") an der Inkubatorkammer beweglich montierte Inkubatortüre, insbesondere eine oder mehrere Kammertüren. Ein Inkubator kann eine oder mehrere Innentüren aufweisen, die insbesondere transparent sein können, und kann eine - insbesondere nicht transparente- Außentür aufweisen, welche insbesondere die Inkubatorkammer und gegebenenfalls mindestens einer innere Inkubatortüre, welche die Kammeröffnung verschließt bzw. öffnet, zur Umgebung hin thermisch isoliert.

In der verschlossenen Position der Kammeröffnung ist das Innere der Inkubatorkammer gegen die Umgebung vorzugsweise in der Weise isoliert, so dass im Inneren eine gewünschte, vom Inkubator gesteuerte Temperatur bzw. Atmosphäre einstellbar, insbesondere regelbar ist. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in einer die Kammeröffnung umlaufenden Frontwand des Inkubators.

Die Inkubatorkammer weist vorzugsweise mehrere Wände bzw. Innenwandflächen auf, die insbesondere einstückig und insbesondere kantenfrei miteinander verbunden sein können. Die Wände bzw. Innenwandflächen sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände bzw. Innenwandflächen sind vorzugsweise aus einem korrosionsarmen Material gefertigt, insbesondere Edelstahl, Kupfer, Messing, oder ein Kunststoff, insbesondere ein Verbundkunststoff. Dadurch wird die Reinigung/ Desinfektion des Kammerinneren erleichtert. Unabhängig von der Kammeröffnung, die dem Bestücken / Entnehmen von Objekten bzw. Zellkulturbehältern dient, kann die Inkubatorkammer mindestens einen Port zum Durchführen einer entsprechend dimensionierten Vorrichtung bzw. einer Kabelverbindung aus dem Inneren der Inkubatorkammer an deren Außenseite oder in die Umgebung des Inkubators aufweisen.

Eine Seitenwand der Inkubatorkammer weist insbesondere eine Dampfeintrittsöffnung auf, die mit der Dampfaustrittsöffnung der Verdampfervorrichtung verbunden ist, insbesondere fluiddicht. Diese Seitenwand der Inkubatorkammer weist insbesondere auch einen Befestigungsabschnitt zur Befestigung der Verdampfervorrichtung auf. Der Befestigungsabschnitt kann die Dampfeintrittsöffnung beinhalten, in der vorzugsweise ein Verbindungselement zur Verbindung der Verdampfervorrichtung mit der Seitenwand angeordnet ist.

Eine typische Größe des Inneren einer Inkubatorkammer liegt zwischen 50 und 400 Litern (dm³).

Das Laborgerät, bzw. der Inkubator, kann genau eine Inkubatorkammer aufweisen, kann aber auch mehrere Inkubatorkammern aufweisen, deren Luftfeuchte der Atmosphäre (oder: Temperatur, relative Gaskonzentration) insbesondere individuell oder gesammelt einstellbar sein kann. Ein Laborgerät kann mehrere Inkubatorkammern aufweisen, die jeweils eine eigene Kammeröffnung und eine eigene Kammertüre zum Verschließen der Kammeröffnung aufweisen können. Jede dieser Inkubatorkammern, oder eine Gruppe dieser Inkubatorkammern, kann mit einer Beleuchtungseinrichtung versehen sein.

Das Laborgerät, bzw. der Inkubator, kann ein Gehäuse aufweisen, das die Inkubatorkammer teilweise oder vollständig umgibt. Eine erfindungsgemäße Beleuchtungseinrichtung ist vorzugsweise, insbesondere neben der Inkubatorkammer, innerhalb des Gehäuses angeordnet. Das Gehäuse kann im Wesentlichen quaderförmig ausgebildet sein, und kann insbesondere derart ausgebildet sein, dass das Laborgerät stapelbar ist.

Das Laborgerät, bzw. der Inkubator, weist vorzugsweise eine Trägereinrichtung auf, die insbesondere eine erste Rahmenseitenwand und, dieser gegenüberliegend, eine zweite Rahmenseitenwand aufweisen kann. Die Beleuchtungseinrichtung erstreckt sich vorzugsweis von außerhalb der zweiten Rahmenseitenwand, insbesondere durch eine thermische Isolationsschicht, bis zur Eintrittsöffnung in der Kammerwand.

Das Laborgerät weist vorzugsweise eine Benutzerschnittstelleneinrichtung (auch bezeichnet als Benutzerschnittstelle) auf. Diese erlaubt die Erfassung von Benutzereingaben über mindestens ein Eingabemittel der Benutzerschnittstelle, insbesondere über einen Touchscreen oder Tastenelemente, und vorzugsweise die Ausgabe von Informationen über ein Display, insbesondere Touchscreen. Die Benutzerschnittstelleneinrichtung ist insbesondere mit der Steuereinrichtung zum Austausch von Signalen, insbesondere Daten, verbunden.

Weitere bevorzugte Ausgestaltungen der erfindungsgemäßen Gegenstände, insbesondere des erfindungsgemäßen Verfahrens, lassen sich aus der Beschreibung des erfindungsgemäßen Laborgeräts mit Inkubationsfunktion und deren bevorzugten Ausgestaltungen entnehmen. Ferner ergeben sich weitere Ausgestaltungsoptionen der Erfindung aus den Ausführungsbeispielen in den Figuren. Gleiche Teile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1a zeigt eine perspektivische seitlich-frontale Ansicht eines erfindungsgemäßen Laborgeräts gemäß Ausführungsbeispiel.
Fig. 1b zeigt das Laborgerät der Fig. 1a, mit Blick in den Kammerinnenraum bei geöffneter Schwenktüre.
Fig. 1c zeigt eine seitlich-perspektivische Ansicht des Laborgeräts der Fig. 1a, mit abgenommener Seitenwand und Sicht in den Elektronikraum.
Fig. 2 zeigt eine geschnittene, seitlich perspektivische Ansicht des Laborgeräts der Fig. 1c, mit Schnitt entlang einer Längsachse A der Beleuchtungseinrichtung des Laborgeräts der Fig. 1c.
Fig. 3a zeigt eine geschnittene, seitlich perspektivische Ansicht der Beleuchtungseinrichtung des Laborgeräts der Fig. 1c.
Fig. 3b zeigt eine seitlich perspektivische Ansicht der Beleuchtungseinrichtung des Laborgeräts der Fig. 1c.
Fig. 4 zeigt ein Detail der Beleuchtungseinrichtung des Laborgeräts der Fig. 1c.
Fig. 5a zeigt eine Frontansicht eines Details der Beleuchtungseinrichtung der Fig. 3b.
Fig. 5b zeigt eine geschnittene, seitlich perspektivische Ansicht eines Details der Beleuchtungseinrichtung des Laborgeräts der Fig. 1c.
Fig. 6a und 6b zeigen jeweils eine geschnittene, seitlich perspektivische Ansicht eines Details des Laborgeräts der Fig. 1c, mit Schnitt entlang einer Längsachse A der Beleuchtungseinrichtung des Laborgeräts der Fig. 1c.

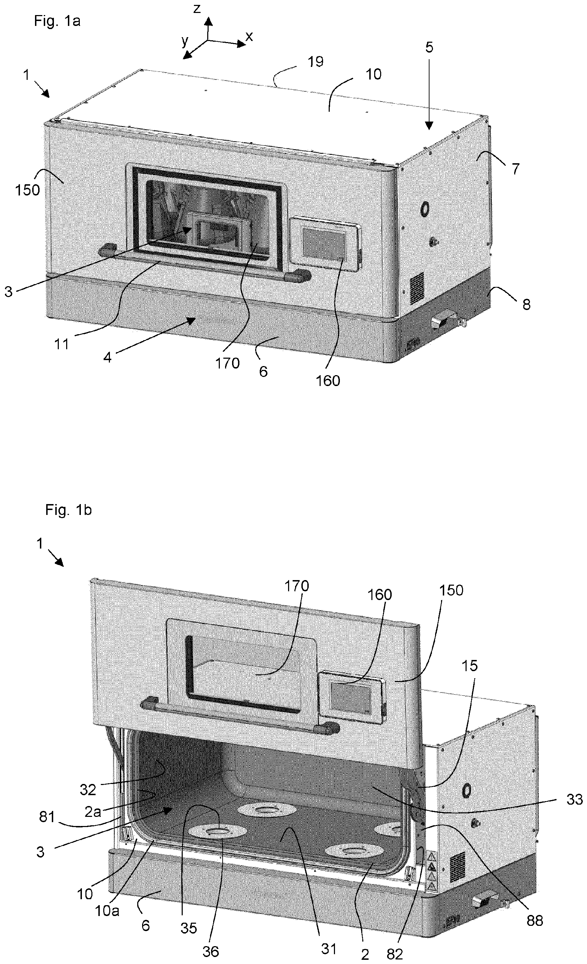

Fig. 1a zeigt eine perspektivische seitlich-frontale Ansicht eines erfindungsgemäßen Laborgeräts 1 mit Inkubationsfunktion, hier eines Laborschüttlers 1. Es handelt sich um einen insbesondere stapelbaren, CO2-Inkubationsschüttler mit beleuchtbarem Kammerinnenraum, der insbesondere ein erfindungsgemäßes Verfahren zur Hochtemperatur-Desinfektion des Kammerinnenraums 3 implementiert. Dies wird insbesondere dadurch ermöglicht, dass ein Lichtleiter anstelle der Lichtquelle selber zur Eintrittsöffnung für das Licht geführt ist. Da der Lichtleiter kein elektronisches Bauteil ist, weist er eine höhere Temperaturunempfindlichkeit auf als die Lichtquelle. Die gemeinsame Anordnung der hocherhitzbaren Kammer und des lichtemittierenden Bauteils wird dadurch erst ermöglicht. Es handelt sich um eine Inkubationskammer 2 mit für die zu inkubierenden Proben passender Atmosphärenzusammensetzung, hier mit definierter CO₂-Konzentration, Temperatur und Luftfeuchte zur Inkubation lebender Zellkulturen.

Der Laborschüttler 1 weist ein Gehäuse 19 auf, in dem die Kammer 2 angeordnet ist. Das Gehäuse 19 weist die Seitenwand 7 auf, eine weitere Seitenwand (nicht gezeigt), eine Rückwand (nicht gezeigt), eine Deckenwand 10, ein Bodenblech (nicht gezeigt), die Frontblende 6, eine rückseitige Blende (nicht gezeigt) und Seitenblenden 8. Die Kammeröffnung 2a der Kammer 2, wie in Fig. 1b gezeigt, ist bei geschlossener Schwenktüre 150 mittels Türdichtung 10a so abgedichtet, dass im Betrieb des Laborschüttlers 1 nur ein vernachlässigbarer Austausch von Gasen oder Wasserdampf zwischen Kammerinnenraum 3 und der Umgebung des Laborschüttlers stattfindet.

Die Schwenktüre weist ein Sichtfenster 170 auf, das als doppelwandige Glasplatte ausgeführt ist. Die Schwenktüre sowie das Sichtfenster sind beheizt, um Kondensation an der Türe zu vermeiden. Die Schwenktüre 150 ist durch eine Schwenkmechanik 15 mit zwei, das Öffnen unterstützenden Gaszugfedern von der Frontwandebene nach oben heraus schwenkbar, die geöffnete Position der Schwenktüre ist in Fig. 1b gezeigt. Beim Schließen der Schwenktüre mittels Griff 11 spannt der Nutzer die Gaszugfedern, was durch das Gewicht der Türe unterstützt wird. Die Schwenktüre trägt ein Touchscreen-Display 160 zur Anzeige von Betriebsparametern und zur Eingabe von benutzerdefinierbaren Parametern. Trotz der relativ hohen Masse der Schwenktüre, die durch die zahlreichen Funktionen der Türe bedingt ist, ist die Bedienung komfortabel.

Die Kammer ist einstückig aus Edelstahl gefertigt, und beinhaltet Seitenwände 32, 34, eine Deckenwand (nicht gezeigt), die Rückwand 33, die Kammerbodenwand 31, sowie ein die Kammeröffnung 2a umlaufendes Frontblech.

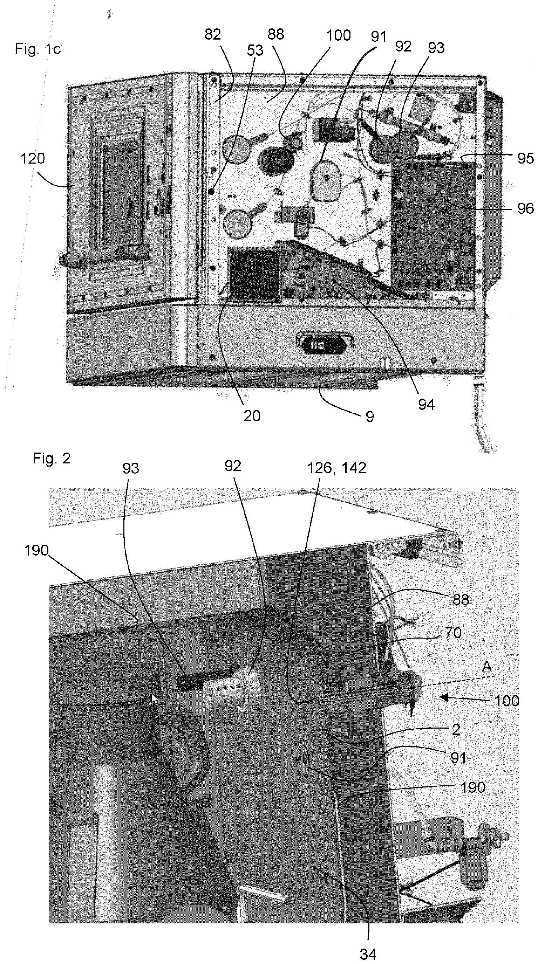

In Fig. 1c ist der Elektronikraum des Laborschüttlers gezeigt, der seitlich der Kammer angeordnet ist. Die Beleuchtungseinrichtung 100 sowie weitere Komponenten befinden sich im Elektronikraum 5. Insbesondere die Türmechanik 15 und der Schüttelantrieb 20, hier ein BLDC-Motor, sind außerhalb der Kammer 2 angeordnet. Dadurch wird der Kammerinnenraum 3 effizient nutzbar, insbesondere werden die Antriebskomponenten während der Durchführung eines auf den Kammerinnraum angewandten Hochtemperatursterilisierungsverfahrens nicht mit erhitzt. Ein Großteil der Antriebsvorrichtung mit Getriebe befindet sich im Vorrichtungsraum 4, der unterhalb des Kammerbodens 31 hinter der Frontblende 6 angeordnet ist. Mittels aus den Bodenöffnungen 35 aus der Kammerbodenwand 31 in den Kammerinnenraum ragenden Kopplungsstangen (nicht gezeigt) wird die zu schüttelnde Plattformeinrichtung (nicht gezeigt) geschüttelt. Die Kammeröffnungen sind dabei durch insbesondere elastische Dichtungselemente (nicht gezeigt) abgedichtet, die mit den Kopplungsstangen verbunden sind und mit diesen während der Schüttelbewegung horizontal bewegt werden, während die Dichtungselemente dicht an der Gleitfläche 36 anliegen und auf dieser gleiten.

Verschiedene im Elektronikraum gezeigte elektronische Komponenten sind an der Rahmenwand 88 angeordnet, insbesondere die Beleuchtungseinrichtung 100, der Verdampfer 91, der CO₂-Sensor 92, der Luftfeuchtesensor 93, die Spannungsversorgungskomponenten 94 des Antriebs und der Heizeinrichtung zum Beheizen der Kammer, sowie die Elektronikplatine 95. Die Elektronikplatine 95 beinhaltet insbesondere die Steuerungseinrichtung 96 des Laborschüttlers. Diese ist insbesondere dazu programmiert, ein auf den Kammerinnraum 3 angewandtes Hochtemperatursterilisierungsverfahren, gemäß einem Aspekt der Erfindung, auszuführen. Sichtbar ist ebenso der Wasserverdampfer 25, mit dem Wasser verdampfbar und in den Kammerinnenraum einleitbar ist.

Mittels des Türsensors 53 registriert die Steuereinrichtung 96 ein Öffnen und/oder Schliessen der Türe 150, insbesondere eine Aktivität der Schwenkmechanik 15. Die Steuereinrichtung ist programmiert, bei einem Öffnen der Türe die Beleuchtung des Kammerinnenraums mittels der Beleuchtungseinrichtung 100 einzuschalten, insbesondere mittels Fadein der Beleuchtungsintensität, und bei einem Schliessen der Türe die Beleuchtung des Kammerinnenraums auszuschalten, insbesondere mittels Fade-out der Beleuchtungsintensität. Die Beleuchtungsfarbe ist dann z.B. weiß.

Getragen werden die Komponenten des Laborschüttlers von einer ersten seitlichen Rahmenwand (nicht gezeigt), und dieser gegenüberliegend, die zweite seitliche Rahmenwand 88. Die beiden Rahmenwände stehen auf der Gerätebasis 9 und sind mit dieser fest verbunden. Durch die Gehäusedeckenwand 10 und die Gehäuserückwand ist die Trägereinrichtung 88 weiter stabilisiert.

Fig. 2 zeigt einen Schnitt durch die Beleuchtungseinrichtung 100, entlang deren Längsachse, die senkrecht zur Kammerseitenwand 34 angeordnet ist. Die Blickrichtung der Figur lässt die Innenseite der Kammerseitenwand 34 erkennen, und zeigt die Positionen
i) einer kombinierten Eintrittsöffnung 126, 142 für Licht und CO₂-Gas;
ii) einer Eintrittsöffnung für den Dampf, der aus dem Verdampfer 91 austritt und in den Kammerinnenraum eintritt;
iii) des CO₂-Sensors 92;
iv) des Luftfeuchtesensors 93.

Die genannten Komponenten sind zudem in der Weise senkrecht zur Kammerseitenwand 34 angeordnet, dass sie von der Außenseite der Rahmenseitenwand 88 durch Öffnungen der Rahmenseitenwand 88 treten, durch Öffnungen der thermischen Isolationsschicht 70 geführt sind und schließlich durch Öffnungen in der Kammerseitenwand 34 in den Kammerinnenraum treten, wobei diese Öffnungen in der Kammerseitenwand 34 durch Abdichtmittel gasdicht abgedichtet sind. Die thermische Isolationsschicht besteht vorzugsweise aus einem porösem Kunststoff oder weist diesen auf. Es kann aber auch Glas- und/oder Mineralwolle verwendet werden.

Die Beleuchtungseinrichtung 100 hat gleichzeitig die Funktion einer Gasleitung (hier: Gaszufuhr, hier für CO₂-Gas), und bildet somit eine kombinierte Gas- und Licht-Führungseinrichtung. Dies wird nachfolgend erläutert.

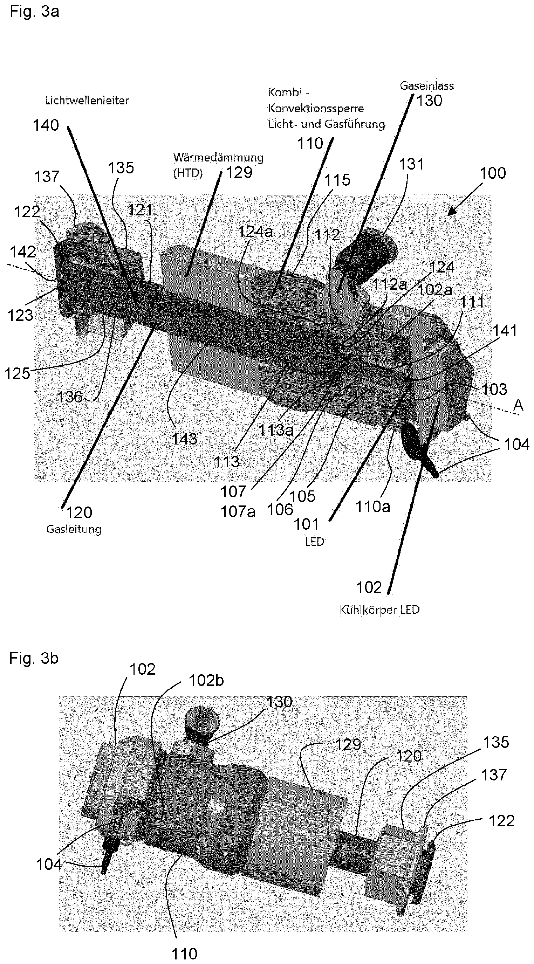

Fig. 3a zeigt die Beleuchtungseinrichtung 100. Diese wird hier durch eine kombinierte Gas- und Licht-Führungseinrichtung 100 gebildet. Diese Komponente 100 kombinierte Gasführungs- und Beleuchtungseinrichtung ist auch dann funktionsfähig, wenn eine der beiden Funktionen ausfällt oder ungenutzt bleibt. Die Komponente 100 kann als Beleuchtungseinrichtung mit Gasführungsfunktion oder als Gasführungseinrichtung mit Beleuchtungseinrichtung beschrieben werden.

Gehalten wird die Komponente 100 primär durch eine als Klemmeinrichtung 122, 137 ausgestaltete Befestigungseinrichtung. Diese wird hier realisiert durch den Flansch 137 einer Mutter 135 und den Flansch 122 einer Halteeinrichtung 120, deren hohlzylinderförmiger Basiskörper 121 gleichzeitig als Gasleitung dient. Das Innengewinde 136 der Mutter 135 wird so auf ein Außengewinde 125 des hohlzylinderförmigen Basiskörpers 121 aufgeschraubt, dass die Kammerwand 34 zwischen dem Flansch 137 der Mutter und dem Flansch 122 der Halteeinrichtung 120 festgeklemmt wird. Zudem weist die kombinierte Gas- und Licht-Führungseinrichtung 100 ein Anschlusselement 110 auf, in dessen zentrale hohlzylindrische Aussparung 113 mit dem Innengewinde 113a das dem Flansch 122 gegenüberliegende Ende des hohlzylinderförmigen Basiskörpers 121 eingeschraubt ist, das dort das Außengewinde 124a aufweist. Dadurch ist das Anschlusselement 110 über die Halteeinrichtung 120 ebenfalls fest mit der Kammerwand 34 verbunden. Das Anschlusselement besteht, wie der Rest der Halteeinrichtung, aus Kunststoff, insbesondere PEEK.

Kernbestandteile der Beleuchtungseinrichtung sind die LED 101 als Lichtquelle und der hier lineare Lichtleiter 140 als Lichtführungseinrichtung, der sich entlang einer zentralen Längsachse A erstreckt. Die LED 101 ist so angeordnet, dass ihre Richtung der Lichtemission senkrecht zu einer ersten Stirnseite 141 des Lichtleiters 140 angeordnet ist. Insbesondere berührt die LED 101 diese Stirnseite 141. Der kappenförmige Kühlkörper 102, der gleichzeitig als Halteelement für die LED 101 dient, wird über ein Innengewinde 102a an der inneren Randseite seines Kappenkörpers auf das passende Außengewinde 110a aufgeschraubt, das konzentrisch zur Achse A liegt. Auch der hohlzylinderförmige Basiskörper 121 der Halteeinrichtung und das bis auf die Gasanschlussbuchse 112 im Wesentlichen rotationssymmetrische Anschlusselement 110 sind konzentrisch zur Achse A. Die Bauform des Lichtleiters 140 als lineares, zur Kammerwand 34 senkrecht angeordnetes, Element bestimmt somit die Gestaltung der Komponenten 100.

Die Gasleitung 131 ist von einer Anschlussstelle auf der Außenseite des Gehäuses bis zu dem Gaseinlasselement 130 geführt. Dieses mündet, schräg bzw. hier senkrecht zur Achse A angeordnet, in der Gasanschlussbuchse 112 des Anschlusselements 110. Möglich wäre aber unter anderem auch eine andere, hier nicht realisierte konstruktive Variante, bei der die Gasleitung konzentrisch und koaxial am Ende 124 des als Halteelement dienenden Gasleitungselements 120 mündet, und ein gekrümmter Lichtleiter durch eine, hier nicht vorhandene, seitliche Öffnung des Gasleitungselements 120 gasdicht geführt wird.

Das Anschlusselement weist im Wesentlichen drei Anschlüsse 111, 112, 113 auf. Der Anschluss 111 ist eine hohlzylindrische Aussparung 111, die koaxial zur bereits erläuterten hohlzylindrischen Aussparung 113 am anderen Ende des Anschlusselements angeordnet ist. Die hohlzylindrische Aussparung 111 nimmt formschlüssig eine Hülse 105 auf, die wiederum als Führung für den stabförmigen Lichtleiter 140 dient. Die hohlzylindrischen Aussparung 113 am ersten Ende des Anschlusselements 110 und die hohlzylindrischen Aussparung 113 am zweiten Ende des Anschlusselements 110 sind über eine hohlzylindrisch Aussparung 107 verbunden, deren Durchmesser jeweils kleiner ist als der Durchmesser der beiden Aussparungen 111 und 113, deren Durchmesser aber zur formschlüssigen Aufnahme des stabförmigen Körpers 143 des Lichtleiters 140 geeignet ist.

Zwischen einer ersten Stirnseite der Hülse 105 und einem kreisringförmigen Bodenabschnitt der Aussparung 111 ist ein O-förmiger, elastischer Dichtring 106 angeordnet. In der montierten Position drückt die Platine 103 die Hülse 105 gegen den Bodenabschnitt der Aussparung 111, wodurch der O-Ring 106 verformt wird und gegen den Lichtleiter 140 gepresst wird. Dadurch wird der zylindermantelförmige Spaltdurchgang zwischen Lichtleiter und Aussparung 107 abgedichtet. Das ist nötig, da dieser zylindermantelförmige Spaltdurchgang 107a als Gaseinlassabschnitt 107a dient. Noch relevanter ist, dass andernfalls die feuchte Luft aus der Inkubatorkammer zur LED diffundieren könnte. Dort könnte es zu Korrosion und in Folge dessen zum Ausfall der Beleuchtung kommen. Der Gaseinlasskanal 112a mündet am ersten Ende in der Anschlussbuchse 112 und am zweiten Ende im Gaseinlassabschnitt 107a. Der Gaseinlassabschnitt ist hülsenseitig abgedichtet und mündet am anderen Ende in den zylinderförmigen Hohlraum des hohlzylinderförmigen Basiskörpers 121 der Halteeinrichtung, der als Gasleitung dient. Die Gewindeverbindung 124a, 113a zwischen Halteelement 120 und Anschlussbuchse 113 ist gasdicht.

Die LED 101 der Beleuchtungseinrichtung ist auf einer Platine 103 befestigt, welche von einem Kühlkörperteil 102 gekühlt wird. Aus dem Kühlkörperelement 102 führen seitlich die Spannungsversorgungskabel 104 für die LED 101 heraus, die im Inneren des Kühlkörperelements 102 mit Anschlussstellen (nicht gezeigt) auf der Platine 103 verbunden sind. Figur 3b zeigt, dass der kappenförmige Körper 102 zwei schlitzförmige Aussparungen aufweist. Diese sind durch die Kabelenden abgedichtet.

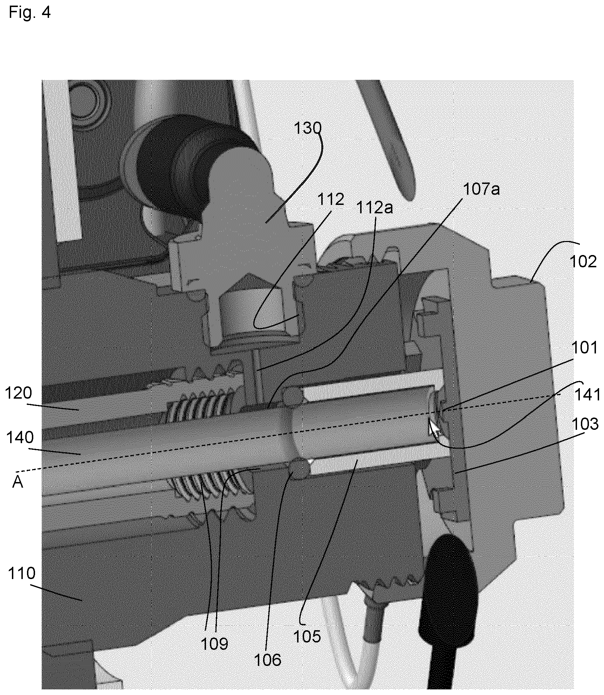

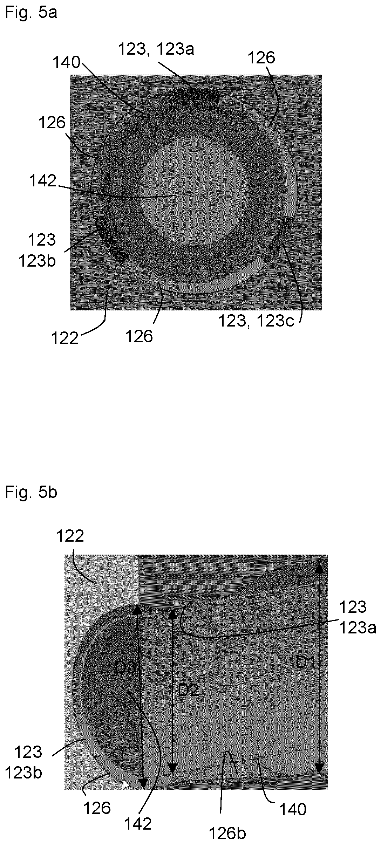

Fig. 4 zeigt in vergrößerter Ansicht, wie der Gaseinlasskanal 112a senkrecht zur Achse A in den Gaseinlassspalt 107a mündet. Von dort gelangt das Gas in den Gasleitungsraum 109, wird durch den mantelförmigen Hohlraum zwischen Lichtleiter und Hohlzylinderinnenwand des Halteelements 121 in Richtung der Gasaustrittsöffnung 126 geführt. Diese ist in Fig. 5a gezeigt.

Fig. 5a zeigt eine Frontansicht aus axialer Richtung auf den Flansch 122 der Halteeinrichtung 120 und die Stirnseite 142 des Lichtleiters 140, welche in den Kammerinnenraum 3 mündet. In die kreisförmige Austrittsöffnung 126 mündet der Lichtleiter 140. Dessen Stirnseite 142 ist im Wesentliche koplanar mit der Außenfläche des Flansches 122, kann aber auch aus der Gasaustrittsöffnung 126 in den Kammerinnenraum 3 hineinragen, oder kann nicht bis zur Ebene der Gasaustrittsöffnung 126 reichen.

Fig. 5a und 5b zeigen, dass sich der zylinderförmige Hohlraum des Haltelements 120 den Durchmesser D1 aufweist und sich Richtung Auslassöffnung 126 zu einer Engstelle 126b verjüngt, deren geringster Durchmesser D2 kleiner ist als der Durchmesser D3 der Auslassöffnung, hier: D2 < D3 < D1. In der Engstelle sind zudem die von der Innenseite der Engstelle radial nach innen weisenden Vorsprünge 123a, 123b, 123c entlang der kreisförmigen Umfangsrichtung der Innenseite gleichmäßig verteilt. Diese drei Vorsprünge dienen als Klemmeinrichtung, um den Lichtleiter nahe der Auslassöffnung 126 zu fixieren. Beim Einsetzen des Lichtleiters verdrängt die Stirnseite 142 des Lichtleiters abrasiv eine Materialschicht der Vorsprünge, so dass der Lichtleiter form- und/oder kraftschlüssig zwischen den Vorsprüngen gehalten wird. Zusammen mit der Klemmung durch den O-Ringe 106 am anderen Ende des Lichtleiters wird dieser ausreichend fixiert, um einerseits ein Bewegen in radialen Richtungen zu verhindern und andererseits ein Verrutschen in axialer Richtung zu verhindern.

Fig. 5b zeigt eine geschnittene, seitlich perspektivische Ansicht eines Details der Beleuchtungseinrichtung des Laborgeräts der Fig. 1c.

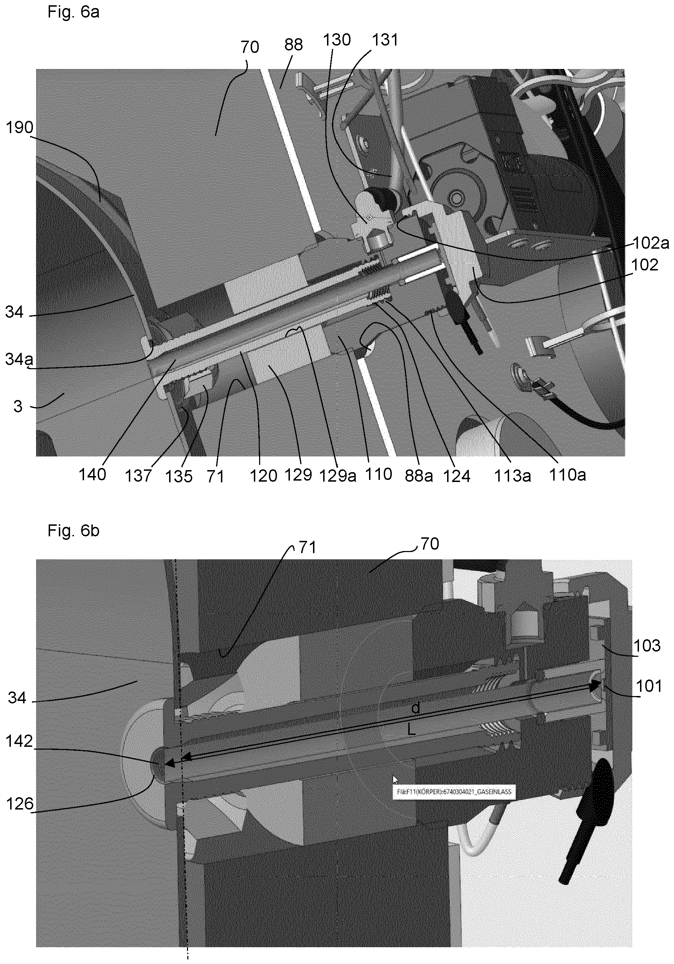

Fig. 6a und 6b zeigen jeweils eine geschnittene, seitlich perspektivische Ansicht eines Details des Laborgeräts der Fig. 1c, mit Schnitt entlang einer Längsachse A der Beleuchtungseinrichtung des Laborgeräts der Fig. 1c.In Fig. 6a ist gezeigt, dass das Halteelement 120 aus Richtung des Kammerinnenraums 3 in die Bohrung 34a der Kammerseitenwand 34 eingesetzt ist. Das Halteelement 120 führt formschlüssig durch eine Durchgangsöffnung 129a eines thermisch isolierenden Schaumstoffstopfens 129, insbesondere aus porösem Kunststoff, insbesondere Polyisocyanurat, der in die Durchgangsöffnung 71 der thermischen Isolationsschicht 70 eingesetzt ist. Das aus Kunststoff bestehende Anschlusselement 110 weist einen Abschnitt 115 auf, dessen Durchmesser etwas größer ist als der Durchmesser der Durchgangsöffnung 71 der thermischen Isolationsschicht 70. Dadurch verdrängt das Anschlusselement 110 beim Einsetzen in die Durchgangsöffnung 71 das elastische Material der thermischen Isolationsschicht 70, wodurch es kraftschlüssig gehalten wird und als Konvektionssperre dient. Dagegen tritt das Anschlusselement 110 hier berührungsfrei durch die Öffnung 88a der Rahmenseitenwand 88. Die Komponente 100 wird deshalb im Wesentlichen durch die Befestigungseinrichtung 122, 137 an der Kammerseitenwand 34 gehalten, stützt sich aber auch am thermischen Isolationsmaterial 70 ab. Das Anschlusselement 110 kann aber auch an der Rahmenseitenwand 88 montierbar sein bzw. montiert sein.

Figur 6b zeigt insbesondere die Stirnseite 142 des Lichtleiters 140. Der Lichtleiter 140 ist ein Glasstab, insbesondere aus temperaturbeständigem Borosilikatglas, dessen Stirnseiten insbesondere durch Abbrechen eines Glasstabes entstehen und hier insbesondere weder poliert noch linsenförmig sind. Die dadurch entstehende Beleuchtungswirkung zur Beleuchtung des Innenraums 3 erfüllt die Anforderungen.

Durch die kombinierte Gas- und Lichteintrittsöffnung ist nur eine Bohrung 34a in der Kammerseitenwand 34 (und ebenso der Rahmenseitenwand 88) erforderlich, was potentiell die Anzahl der abzudichtenden Öffnungen der Kammer 2 reduziert und damit auch das Risiko von unnötiger Leckage. Durch die Länge des Lichtleiters, die hier L = 9,7 cm beträgt, ist die Lichtquelle 101 ausreichend weit von der Kammer entfernt, wenn diese mithilfe der Heizwendel in einem Hochtemperaturverfahren für einen Zeitraum zwischen 5 min und 4 h auf 180 °C erhitzt wird. Der Lichtleiter 140 ist temperaturbeständig und kann im Gegensatz zu einer LED 101 nahe an die erhitzte Kammer 2, bzw. die Kammerseitenwand 34 herangeführt werden. Grundsätzlich bietet ein Lichtleiter als Lichtemitter in der Kammer oder an der Kammerwand den Vorteil, dass die Lichtquelle nicht an der Kammer positioniert werden muss sondern an beliebiger Stelle im Laborgerät positioniert werden kann. Zudem ist die Kammer einfacher zu reinigen.

## Patentansprüche

1. Laborgerät zur Inkubation von in Probengefäßen (130) enthaltenen flüssigen Laborproben, insbesondere Inkubationsschüttler, aufweisend
eine Inkubationskammer (2), die mindestens eine Kammerwand aufweist und eine Kammeröffnung (2a) zum Einstellen und Herausnehmen der Probengefäße (130) in einen Innenraum (3) der Kammer,
eine Heizeinrichtung (190), die zum Beheizen der Inkubationskammer vorgesehen ist,
eine Beleuchtungseinrichtung (100) zum Beleuchten des Innenraums (3) der Inkubationskammer,
**dadurch gekennzeichnet, dass**
die mindestens eine Kammerwand (34) mindestens eine Eintrittsöffnung (34a) aufweist, und
die Beleuchtungseinrichtung (100) mindestens eine Lichtquelle (101) und mindestens einen Lichtleiter (140) aufweist, der dazu angeordnet ist, das von der mindestens einen Lichtquelle (101) emittierte Licht zu der mindestens einen Eintrittsöffnung (34a) zu führen und den Innenraum (3) der Inkubationskammer zu beleuchten.

2. Laborgerät gemäß Anspruch 1, wobei eine Lichtquelle (101) in einem Abstand (d) zu einer Kammerwand (34) angeordnet ist, und ein Lichtleiter (140) sich ausgehend von der Lichtquelle (101) bis zu einer Eintrittsöffnung (34a) erstreckt, insbesondere entlang einer Längsachse (A), und/oder insbesondere senkrecht zur Kammerwand (34) verläuft.

3. Laborgerät gemäß Anspruch 1 oder 2, wobei die Beleuchtungseinrichtung (100) eine Halteeinrichtung (120, 110, 105, 106) aufweist, die den Lichtleiter (140) relativ zur Eintrittsöffnung (34a) in einer Montageposition hält, und die insbesondere mindestens ein Haltemittel (122; 135) aufweist, mit dem der Lichtleiter (140) an der Eintrittsöffnung (34a) gehalten wird.

4. Laborgerät gemäß mindestens einem der vorangehenden Ansprüche, wobei an der Außenseite der Kammerwand (34) eine thermische Isolationsschicht (70) angeordnet ist, die eine Öffnung (71) aufweist, durch die sich der Lichtleiter (140) bis zur Eintrittsöffnung (34a) erstreckt.

5. Laborgerät gemäß Anspruch 3 und einem der vorangehenden Ansprüche, wobei die Halteeinrichtung (120, 110, 105, 106) ein Haltemittel (129; 110) aufweist, das mit der thermischen Isolationsschicht verbunden ist, insbesondere formschlüssig und/oder kraftschlüssig verbunden ist.

6. Laborgerät gemäß mindestens einem der vorangehenden Ansprüche, das mindestens ein Gasleitungselement (120) aufweist, die in die Eintrittsöffnung (34a) mündet, so dass ein aus der Gasleitungselement (120) strömendes Gas in den Kammerinnenraum (3) gelangt.

7. Laborgerät gemäß Anspruch 6, wobei der Lichtleiter (140) und das Gasleitungselement (120) entlang eines gemeinsamen Pfades (A) verlaufen und in derselben Eintrittsöffnung (34a) münden, wobei vorzugsweise der Lichtleiter (140) und das Gasleitungselement (120) koaxial verlaufen.

8. Laborgerät gemäß einem der Ansprüche 6 oder 7, wobei der Lichtleiter (140) innerhalb des Gasleitungselements (120) verläuft und in einer Auslassöffnung (126) des Gasleitungselements (120) mündet.

9. Laborgerät gemäß Anspruch 8, wobei der Lichtleiter (140) form- und/oder kraftschlüssig in der Auslassöffnung (126) gehalten wird.

10. Laborgerät gemäß Anspruch 9, wobei ein Innenraum des Gasleitungselements (120), insbesondere die Auslassöffnung (126), mindestens einen radial nach innen weisenden Vorsprung (123; 123a; 123b; 123c), vorzugsweise drei oder mehr solcher Vorsprünge, aufweist, an dem sich der Lichtleiter (140) abstützt.

11. Laborgerät gemäß einem der Ansprüche 6 bis 10, wobei das Gasleitungselement (120) ein hohlzylinderförmiges Bauteil ist, in das der Lichtleiter (140) eingeführt ist, insbesondere an einer Stirnseite (124) des hohlzylinderförmigen Bauteils (120).

12. Laborgerät gemäß einem der Ansprüche 6 bis 11, das ein Anschlusselement (110) aufweist, das insbesondere Teil der Halteeinrichtung (120, 110, 105, 106) ist, und das einen Aufnahmeraum (109) aufweist, in den ein stromaufwärts gelegenes Ende (124) des Gasleitungselements (120) mündet, und in den ein stromabwärts gelegenes Ende einer Gasanschlussleitung (112a) mündet und durch den insbesondere der Lichtleiter (140) verläuft und an dem insbesondere eine Lichtquelle (101) befestigt und angeordnet ist, um Licht in eine Stirnseite (141) des Lichtleiters (140) einzukoppeln.

13. Laborgerät gemäß einem der Ansprüche 11 oder 12, wobei das Gasleitungselement (120) am stromabwärts gelegenen Ende (122) einen Flansch (122) aufweist, der insbesondere Teil der Halteeinrichtung (120, 110, 105, 106) ist, und der innerhalb der Inkubationskammer (2) angeordnet ist und dort an der Eintrittsöffnung (34a) anliegt, während ein hohlzylinderförmiger Abschnitt (121) des hohlzylinderförmigen Bauteils (120) sich durch die Eintrittsöffnung (34a) erstreckt.

14. Laborgerät gemäß mindestens einem der vorangehenden Ansprüche, wobei ein Lichtleiter (140) ein Glasstab ist oder diesen aufweist, und der Glasstab insbesondere aus Borosilikatglas besteht.

15. Verfahren zur Behandlung des Kammerinnenraums eines Laborgeräts gemäß einem der vorangehenden Ansprüche, das eine elektrische Steuereinrichtung (96) aufweist, die dazu programmiert ist, ein Hochtemperaturprogramm auszuführen, so dass der beleuchtbare Kammerinnenraum (3) mittels der Heizeinrichtung (190) für einen vorbestimmten Zeitraum auf eine vorbestimmte Temperatur aufgeheizt wird, wobei der Zeitraum zwischen 1 Minute und 12 Stunden betragen kann, und wobei die Temperatur zwischen 90 °C und 200°C betragen kann, insbesondere zwischen 120 °C und 200 °C, und besonders bevorzugt zwischen einschließlich 180 °C und 200 °C, wobei das Verfahren den Schritt aufweist:
• Aufheizen des beleuchtbaren oder beleuchteten Kammerinnenraums (3) mittels der Heizeinrichtung (190) für einen vorbestimmten Zeitraum auf eine vorbestimmte Temperatur, wobei der Zeitraum zwischens 1 Minute und 12 Stunden betragen kann, und wobei die Temperatur zwischen 90 °C und 200°C betragen kann, insbesondere zwischen 120 °C und 200 °C und besonders bevorzugt zwischen 180 °C und 200 °C, betragen kann.

16. Verfahren zur Beleuchtung des Kammerinnenraums eines Laborgeräts gemäß einem der vorangehenden Ansprüche, das eine elektrische Steuereinrichtung (96) aufweist, die dazu programmiert ist, mindestens eine Funktion der Beleuchtungseinrichtung zu steuern, insbesondere die Dauer bzw. der Zeitverlauf und/oder Intensität und/oder Farbe des eingestrahlten Lichts und/oder in Abhängigkeit von Sensorsignalen, einem Geräteparameter, und/oder einer Nutzereingabe.
